# EUROPEAN PATENT SPECIFICATION

(11) **EP 4 388 084 B1**
(45) Date of publication and mention of the grant of the patent: **11.06.2025**
(21) Application number: 22785937.8
(22) Date of filing: 09.09.2022
(51) Int. Cl.: C12N 9/00, C12Q 1/66, G01N 33/543

(54) **METHOD FOR DETERMINING THE CONCENTRATION OF INORGANIC PYROPHOSPHATE**
VERFAHREN ZUR BESTIMMUNG DER KONZENTRATION VON ANORGANISCHEM PYROPHOSPHAT
PROCÉDÉ POUR DÉTERMINER LA CONCENTRATION DE PYROPHOSPHATE INORGANIQUE

(30) Priority: 09.09.2021 EP 21306235
(43) Date of publication of application: 26.06.2024
(73) Proprietor: Centre National de la Recherche Scientifique, 75016 Paris (FR); Université Côte d'Azur, 06100 Nice (FR); CHU de NICE, 06200 Nice (FR)
(72) Inventor: DURANTON, Christophe, 06100 Nice (FR); RUBERA, Isabelle, 06000 Nice (FR); FAVRE, Guillaume, Alexandre, 06000 Nice (FR); LAURAIN, Audrey, 06000 Nice (FR); LEFTHERIOTIS, Georges, 06950 Falicon (FR)
(74) Representative: IXAS Conseil
(86) International application number: PCT/EP2022/075142
(87) International publication number: WO 2023/036949

(56) References cited:
- US-A1- 2009 317 803
- SILCOX DONALD C. ET AL: "Identification of Inorganic Pyrophosphate in Human Platelets and Its Release on Stimulation with Thrombin", THE JOURNAL OF CLINICAL INVESTIGATION, vol. 52, no. 7, 1 July 1973 (1973-07-01), GB, pages 1595 - 1600, XP055891246, ISSN: 0021-9738, DOI: 10.1172/JCI107336
- MARQUES S M ET AL: "Optimized chromatographic and bioluminescent methods for inorganic pyrophosphate based on its conversion to ATP by firefly luciferase", TALANTA, ELSEVIER, AMSTERDAM, NL, vol. 77, no. 4, 15 February 2009 (2009-02-15), pages 1497 - 1503, XP025780986, ISSN: 0039-9140, [retrieved on 20080927], DOI: 10.1016/J.TALANTA.2008.09.032
- SUTOR D ET AL: "The estimation of pyrophosphate in urine with uridine-5'-diphosphoglucose pyrophosphorylase", CLINICA CHIMICA ACTA, ELSEVIER BV, AMSTERDAM, NL, vol. 86, no. 3, 15 June 1978 (1978-06-15), pages 329 - 332, XP024785021, ISSN: 0009-8981, [retrieved on 19780615], DOI: 10.1016/0009-8981(78)90389-3
- CARTIER P H ET AL: "Measurement of inorganic pyrophosphate in biological fluids and bone tissues", ANALYTICAL BIOCHEMISTRY, ACADEMIC PRESS, AMSTERDAM, NL, vol. 61, no. 2, 1 October 1974 (1974-10-01), pages 416 - 428, XP024828375, ISSN: 0003-2697, [retrieved on 19741001], DOI: 10.1016/0003-2697(74)90407-2
- SIMONET BARTOLOME M ET AL: "Enzymatic determination of pyrophosphate in urine by flow methods", ANALYTICAL SCIENCES, 1 July 2003 (2003-07-01), XP055889534, Retrieved from the Internet <URL:https://www.jstage.jst.go.jp/article/analsci/19/7/19_7_1029/_pdf> [retrieved on 20220209], DOI: 10.2116/analsci.19.1029
- MIWA OHNISHI ET AL: "Inorganic phosphate uptake in intact vacuoles isolated from suspension-cultured cells of Catharanthus roseus (L.) G. Don under varying Pi status", PLANTA ; AN INTERNATIONAL JOURNAL OF PLANT BIOLOGY, SPRINGER, BERLIN, DE, vol. 225, no. 3, 6 September 2006 (2006-09-06), pages 711 - 718, XP019491710, ISSN: 1432-2048

## Description

The present invention relates to determination of the level of inorganic pyrophosphate and more particularly to methods for determining the level of inorganic pyrophosphate in biological samples. The invention also relates to method for *in vitro* detection of a condition associated with abnormal levels of circulating inorganic pyrophosphates.

Inorganic pyrophosphate (PPi) is a critical factor regulating mineral deposition and the biosynthesis of several intracellular compounds. It is now demonstrated that circulating PPi prevents unwanted crystallization of hydroxyapatite in various connective tissues, including vascular wall, kidneys, pericellular bone matrix (see for review Terkeltaub, 2001, Am. J. Physiol., Cell Physiol. 281, C1-C11). In *in vitro* experiments, micromolar of PPi have the ability to inhibit millimolar of calcium and phosphate solutions to form insoluble hydroxyapatite crystals.

Levels of circulating PPi are linked to the activities of several proteins. Several inherited mutations altering the functions of one of these proteins have emphasized the pivotal role of PPi deficiency associated to ectopic tissue mineralisation.

The measurements of PPi levels in biological fluids are now performed since more than 50 years. Different technical approaches have been developed ranging from radioactive, colorimetric, enzymatic and chromatographic techniques. However, the exact concentration of PPi detected in the human plasma is varying strongly between the different studies.

PPi concentrations in biological samples were initially measured using the method of Fiske and Subbarow (Fiske and Subbarow, 1925 J. Biol. Chem. 66, 375-400), that works through the acid hydrolysis of PPi into phosphate and its subsequent quantification. But this method was strongly limited by its low sensitivity and the interactions with the endogenous presence of phosphate.

Quantification of PPi levels in healthy human plasmas using a radioactive method based on isotopic dilution using 32P pyrophosphate reported a plasma PPi concentration ranging from 1.2 up to 5.6 µM with a mean of 3.50 ± 0.11µM (Russell et al., 1971, American Society for Clinical Investigation). A method based on spectrometric quantification through the measurement of reduced phosphomolybdate by yeast pyrophosphatase after removal of phosphate and proteins from the samples (Silcox and McCarty, 1973 J. Clin. Invest. 52, 1863-1870) reported values of 1.80 ± 0.06 µM in healthy patient plasmas (ranging from 0.16 to 3.4 µM). Using a UDPG-pyrophosphorylase enzymatic method coupled to fluorometric measurement of neo-formed NADPH, Lust et al. (Lust and Seegmiller, 1976 Clin. Chim. Acta 66, 241-249) found a PPi concentration of 2.72 ± 0.14 µM. In a seminal study, Ryan et al. (Ryan et al., 1979 Arthritis & Rheumatism 22, 886-891) reviewed the plasma concentration of PPi from several studies and came to conclusion that "the probable existence of one or more uncontrolled variables can be inferred because the mean normal plasma PPi varied from a low of 1.8 µM to a high of 3.5 µM". Later one, Lomashvili et al. described a method using [14C]UDP glucose modified from Lust and Seegmiller and showed a mean plasma level of 3.0 µM of PPi in normal subjects (Lomashvili et al., 2005, J. Am. Soc.Nephrol. 16, 2495 - 2500).

Prosdocimo et al (2009, American Journal of Physiology-Cell Physiology 296, C828-C839.) used an enzymatic method based on the conversion of PPi into ATP, to quantify PPi concentration in biological fluids: the authors reported a concentration of PPi of 1.39 ± 0.30 µM in healthy patients. More recently, using this method, a study performed in human healthy volunteers showed a PPi concentration of ~1.1 µM (Jansen et al., 2014 Arterioscler Thromb Vasc Biol 34, 1985-1989), while a second study showed a basal PPi concentration below the µM range.

The discrepancies of the values for plasma PPi concentration obtained with the different methods strongly limits the comparison of data between the different studies and exclude the possibility to use PPi as a clinical biomarker.

There is therefore a need for a method for determining the concentration of PPi in biological fluids, which is accurate and reproducible.

There is also a need for *in vitro* methods which allow to detect and monitor conditions associated with deregulated PPi levels in an individual, such as mineral and bone disorders and ectopic calcifications.

There is also a need for reliable methods for monitoring the treatment of conditions associated with disorders of mineralization, by measuring a biological marker, which are reproducible during the time.

It has now been found in the context of the present invention that this can be achieved by a technic combining two kinds of technic to quantify the PPi concentration, namely an enzymatic method and an ion chromatography method.

This is why the present invention relates to a method for determining the level of inorganic pyrophosphate (PPi) in a biological sample, comprising :
a) Measuring the concentration of PPi in a first fraction of the biological sample, using an assay based on enzymatic reaction
b) Measuring the concentration of PPi in a second fraction of the biological sample, using an assay based on ionic chromatography
c) Comparing the value measured in step a) and the value measured in step b), and assessing if the difference is above a pre-determined threshold.
d) If the difference between the values measured respectively in step a and in step b is lower or equal to the pre-determined threshold, the concentration of PPi in the biological sample is determined as the mean of said values measured in step a) and in step b).

If the difference between the values measured respectively in step a) and in step b) is greater than the pre-determined threshold, none of the values are considered as corresponding to the concentration of PPi in the biological sample, and the test is considered as false.

Advantageously, if after step c), the difference between the values measured respectively in step a) and in step b) is greater than the pre-determined threshold, steps a) to c) are repeated on new fractions of the biological sample.

Surprisingly, it has been found that a great variability exists in the results obtained by either of the methods, but the use of the two methods to realize cross-control of the values measured for PPi allows to calculate an accurate PPi concentration, corresponding to the real concentration in the biological sample. The PPi is calculated as the average value of the values obtained respectively for each method.

The choice of an appropriate pre-determined threshold for the difference acceptable for the values measured respectively with the 2 technics gives a very good correlation index. It allows to eliminate bias and outlier measures.

According to an embodiment, the pre-determined threshold is equal to 15%.

The method according to the invention is then comprising the following steps :
a) Measuring the concentration of PPi in a first fraction of the biological sample, using an assay based on enzymatic reaction
b) Measuring the concentration of PPi in a second fraction of the biological sample, using an assay based on ionic chromatography
c) Comparing the value measured in step a and the value measured in step b, and assessing if the difference is above 15%
d) If the difference between the values measured respectively in step a and in step b is lower or equal to 15%, the concentration of PPi in the biological sample is determined as the mean of said values measured in step a and in step b.

If the difference between the values measured respectively in step a and in step b is greater than 15%, steps a to c are repeated.

The PPi concentration is preferably expressed as molarity (in microMole/ volume).

The pre-determined threshold will be adapted by the man skilled in the art; it could be for instance equal to 14%, 13%, 12%, 11% or in particular to 10%.

The difference between the values measured by enzymatic assay (V_{enz}) and by assay based on ionic chromatography (V_{ic}) can be calculated as [V_{enz} - V_{ic}] / V_{enz}

With a pre-determined threshold of 15%, it could be necessary to reiterate the measures a) to c) for as much as 30% of the samples assayed.

For a pre-determined threshold of 10%, %, it could be necessary to reiterate the measures a to c for as much as 50% of the samples assayed.

These percentages of conflicting results could vary and are only given as an illustration.

Preferably, the enzymatic assay of step a) is based on the conversion of PPi to ATP in presence of an enzyme, followed by the conversion of the ATP which results in the generation of an optical signal, preferably a chemiluminescent or fluorescent signal.

Preferably, the enzymatic assay of step a) comprises a first step i) wherein PPi is converted to ATP by a reaction catalyzed by the enzyme sulfurylase, more specifically by the enzyme ATP sulfurylase. This step i) is performed in the presence of a nucleotide, more particularly in the presence of adenosine 5' phosphosulfate (APS).

The enzymatic assay of step a) comprises a second step, wherein the de novo-synthetized ATP is subsequently quantified using luciferin/luciferase bio-luminescent assay. The system comprises degradation of ATP to produce light by a luciferin/luciferase system and measurement of light produced. Said reaction is known in the art, and was described for instance by Nyren et al (Anal. Biochem. 151:504, 1985) or Ronaghi et al (Science, 281:363, 1998).

Briefly, ATP drives the luciferase-mediated conversion of luciferin to oxyluciferin that generates visible light in quantities that are proportional to the quantity of ATP. The light produced in the luciferase-catalyzed reaction may be detected, e.g., by a charge coupled device (CCD) camera, photodiode and/or photomultiplier tube (PMT). Light signals are proportional to initial concentration of PPi. Detected signal can be translated into a system output corresponding to the results which is viewable by a user.

Typically, the enzymatic assay of step a) comprises the following steps :
i) converting PPi to ATP by a reaction catalyzed by ATP sulfurylase
ii) producing light from ATP using luciferase in presence of luciferin
iii) detecting the bioluminescence produced as a measure of PPi in the sample.

In a preferred embodiment, a recombinant luciferase protein is used in step ii) in order to generate over the time (at least 10 min) a stable and reliable luminescence. Such protein is commercialized in particular in the ATP Determination Kit ((ATPlite^{®}; PerkinElmer, Waltham, MA) which is used according to the manufacturer's instructions.

In a preferred embodiment of the present invention, a calibration curve is realized by adding standard exogenous PPi solutions to fractions of the biological sample, and performing the assay based on enzymatic reaction, as disclosed here-above, on said fractions containing the exogenous PPi. A standard curve is obtained by measuring the optical signal, and more particularly the bioluminescence generated in media containing increasing concentrations of said exogenous PPi.

It has been evidenced in the present invention, that the composition of the medium can influence the activity of the enzymes (such as ATP-sulfurylase and/or luciferase) involved in the reaction, and therefore alter the reproducibility of the measures. As shown in the present experiments, the matrix (ion, proteins, substance) may alter the measures of enzymatic reaction. As a consequence, bioluminescence measured in different medium, such as water, HBSS or urine, could vary largely for a same PPi concentration, and calibration curves are not directly transposable.

According to the preferred embodiment, a calibration curve is established before measuring the concentration of PPi in the biological sample in the assay based on enzymatic reaction, for each sample. Each biological sample is spiked with increasing concentrations of exogenous PPi (addition of a fresh PPi solution) ; PPi concentration of each biological sample is determined using its own linear regression equation.

In step b), detection of PPi is performed using an ionic chromatography (IC) method. The dosage by IC method combines HPLC method with detectors of ionic conductance (conductimetry). IC method allows to detect significant peaks (retention time around 23 min according to the elution protocol and the column used in this step, see below) which exhibited an increase of their surfaces proportional to the increase of the PPi concentrations.

Calculation of the peaks areas (µs.min-1) measured for the different concentrations of PPi are not significantly different between solutions in water or in HBSS + 10% FBS solution, suggesting that the composition of the matrix has no effect on the PPi concentration using this IC technique.
IC analysis can be performed using chromatography systems known in the art ; for instance an ion chromatography Dionex ICS-5000 plus system (commercialized by Thermo Scientific) can be used. The system includes an autosampler, pumps, eluent generator and conductivity detectors. The system is equipped with an eluent generator (KOH, 500mM), a guard pre-column (AG11-HC, RFIC, 2X50mm) and an analytical anion column (lonPac AS-11-HC, 2X250mm, RFIC) for the detection of PPi.

For PPi measurement the sample is injected and an elution protocol: with KOH followed by a linear increase period is generally performed to reach a stable period before a return period.
The conditions may in a preferred embodiment be the following : Injection loop : 10µl. Elution time 27 min including 0-2min at 7mM KOH, 2-22min gradient starting from 7 mM to 45 mM 22-25 min 50 mM KOH, 25 min to 27 min 7mM

PPi peaks quantification can be performed using adapted software by measuring surface area and compared to the corresponding standard curve.

Preferably, in step b) a calcium chelating agent such as EGTA [ethylene glycol bis(2-aminoethyl ether)-N,N,N',N'-tetraacetic acid] is added to the biological sample before measuring the PPi concentration by the assay based on ionic chromatography. Such calcium chelator is able to complex with calcium ions. Advantageously, the calcium chelating agent exhibits a retention peak in IC which is different from the retention peak of PPi. Calcium chelating agents adapted for the invention are in particular selected among EGTA and derivatives of EGTA.

The method for determining the level of PPi is particularly adapted for biological sample from mammal, such as human or rodents (mouse, rats,...). Biological sample can in particular be selected from blood, plasma, urine, saliva, synovial fluid, CSF (cerebrospinal fluid) and cell culture medium.

In specific embodiments of the method of the invention, the biological sample is submitted to a pre-analytical treatment, before performing steps a) to c).

In particular, the biological sample is submitted to a pre-treatment to remove or reduce the presence of contaminants; said contaminants are for instance selected from proteins, lipids, cells (such as platelets) and cell fragments (erythrocytes, white cells, platelets...). Said contaminants could interfere with the measures of the small concentrations of PPi in the sample. Such pre-treatment is in particular an ultra-filtration.

A pre-treatment adapted to the invention includes, but is not limited to, centrifugation, ultrafiltration and/or congelation. The cut-off for ultrafiltration is selected to eliminate the plasma proteins (for instance albumin, ...) and cells (platelets and monocytes) or fragments of these cells, in particular for blood and plasma samples.

For instance, before ionic chromatography assay, the samples are also submitted to a treatment by an agent inducing precipitation of proteins, such as acetonitrile.

Advantageously, for the enzymatic assay in step a), the bioluminescence produced by the basal ATP level in the biological sample is subtracted from the bioluminescence obtained by addition of ATP sulfurylase; the total bioluminescence observed after adding ATP sulfurylase in the biological sample corresponds to the luciferin/luciferase reaction of both the basal ATP present in the sample and of the ATP generated by conversion of PPi by ATP sulfurylase. To obtain a more accurate measure of PPi in the sample, the bioluminescence obtained in a sample wherein ATP sulfurylase is inactivated, in particular heat inactivated, is measured : the value corresponds to the basal concentration of ATP in the sample.

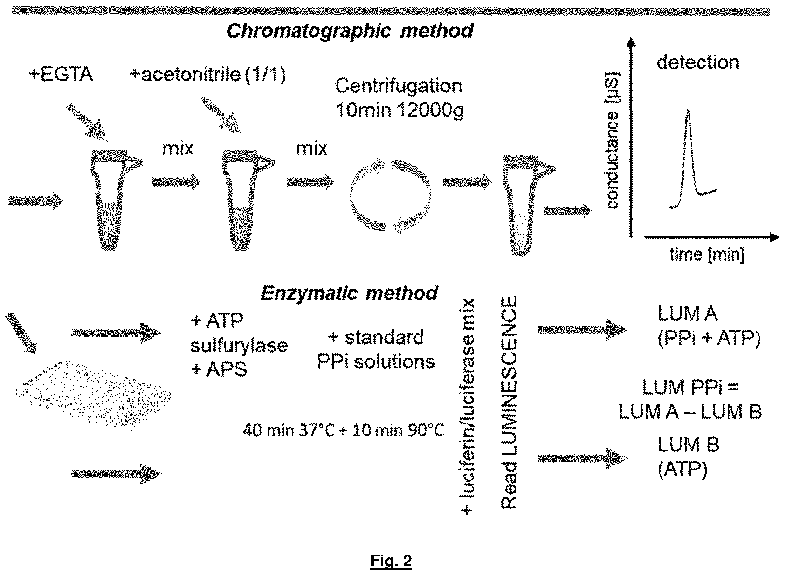

A schematic view of the process is represented on figure 2.

2 solutions are needed. Solution A contains ATPsulfurylase and APS. Solution B containing ATPsulfurylase, is treated to inactivate ATPsulfurylase and then APS is added. Aliquot of each sample or standard are loaded in 2 different wells. In a well Solution A is added. In the second well the same amount of Solution B is added. The plate is then incubated, for instance for 30 min at 37 °C, and subsequently for 10 min at 90 °C, to inactivate ATP sulfurylase. Generated ATP is quantified by luminescence with the use of recombinant luciferase protein. The luminescence (LUM) is measured. PPi values are obtained by subtracting the basal ATP levels measured with solution B.

The invention is also relating to an *in vitro* method for detecting a condition associated with deregulated inorganic pyrophosphate level in an individual, comprising a biological sample from said individual to a method as disclosed above for determining the level of inorganic pyrophosphate (PPi) in said biological sample.

PPi is indeed a physiological inhibitor of mineralization.

ABCC6 mutations cause the inherited disease pseudoxanthoma elasticum (PXE) characterized by mineralization of elastic fibers in some specific tissues. PXE phenotype overlaps with other inherited conditions such as generalized arterial calcification of infancy (GACI, OMIM 208000) or calcification of joints and arteries (ACDC, OMIM 211800). By contrast, loss-of-function mutation(s) in the tnap gene leads to elevated circulating levels of PPi leading to hypophosphatasia (HPP), rickets and chondrocalcinosis. Furthermore, it appears that extracellular PPi homeostasis is a keystone of the ectopic calcification process encountered in several acquired diseases, including CKD (chronic kidney disease), liver diseases and osteodystrophic, rheumatologic and degenerative joint diseases.

Accordingly, the methods of the invention can be used for detecting or monitoring a condition selected in particular among, vascular calcification, pseudoxanthoma elasticum (PXE), hypophosphatasia (HPP), rickets, chondrocalcinosis, chronic kidney disease (CKD), liver diseases, osteodystrophic disease, rheumatologic disease, degenerative joint disease. It can also be used for monitoring the treatment of individuals having one the said conditions, and patients on dialysis and other undetermined pro- or anti-calcifying calcifying pathologies.

The method of the present invention will preferably be used for plasma samples to quantify PPi concentrations in ultrafiltrated plasmas using enzymatic and IC methods in order to permanently cross-control the values measured and to increase the reliability of the result. It allows a reproducible measurement of PPi for the understanding of genetic and metabolic diseases with variable pro- or anti-calcifying phenotypes.

The invention also relates to kits for performing the method as described above. The kit according to the invention comprises ATP sulfurylase, luciferine, luciferase and PPi calibrated solutions, as well as acetonitrile and EGTA ; it may also comprise plasma ultrafilters.

The invention will be better understood with the following examples. The examples are referring to figures
**Figure 1****:** influence of the matrix composition on the measurements of PPi concentration using enzymatic and the IC methods
Figure 1a: Values of luminescence (arbitrary units) measured for increasing concentrations of PPi obtained with the enzymatic bioluminescent method Measurements were performed in 2 different matrix solutions (ultrapure water and HBSS + 10% FBS). PPi concentrations were adjusted to 0.75, 1.5, 3, 5 and 10µM by addition of exogenous PPi from a stock solution (10 mM). Values are means +SEM of 4 different measurements (ANOVA t-test with p ****<0.005).
Figure 1b: Dose response curves measured using the ion chromatographic (IC) method for increasing concentrations of PPi for 2 different matrix solutions (ultrapure water or HBSS supplemented with 10% FBS). The PPi concentrations were fitted to the measured surface of each individual pics. Values are means +SEM of 5 different measurements for each experimental condition.
Figure 1c: Comparison of the PPi concentrations in human urine samples measured using both enzymatic and IC methods
Figure 1d: Comparison of the PPi concentrations measured in the supernatant of WT HEK293 cells or HEK293 cells overexpressing rABCC6 transporter using both enzymatic and IC methods. Values are means +SEM of 4 different measurements.
**Figure 2****:** Description of the different steps for the enzymatic and IC methods used to quantify PPi concentration in human plasmas
**Figure 3****:** Measurements of PPi concentrations in human plasmas and comparison between the enzymatic and IC method
Figure 3a. Illustration of the luminescent slopes measured in PPi spiked ultra-filtrated plasmas.
Figure 3b. Comparison of the PPi plasmatic concentrations (ultrafiltrated plasmas, 80 different donors) measured using both enzymatic and IC methods
Figure 3c. Comparison of the classically used (filters cut-off 300kDa + EDTA) and the modified pre-analytic methods (cut-off 50kDa, no EDTA) using enzymatic quantification of PPi obtained from 42 donors
Figure 3d. Concentrations of PPi measured in the plasmas (ultrafiltrates) of different populations of patients suffering from different diseases known to induce a decrease or an increase of plasmatic PPi concentration.

### Examples

### PPi Calibrating solution

Independent dilutions (from 0.5 to 10 µmol/L) of a 10 mmol/L Sodium pyrophosphate dibasic (PPi, sc-251047 from Santa Cruz Technologies) stock solutions were prepared with ultrapure water (resistivity ≥ 18.2 MΩ/cm, Millipore^{®}, France) or with Hanks' Balanced Salt solution (HBBS, H8264 from Sigma-Aldrich) and used as calibrating solutions.

### Cell culture and cellular PPi release protocol.

The immortalized HEK-293 wild-type and HEK-293 rABCC6 cell lines (kindly provided by Pr. Van de Vetering (Jansen et al., 2013) were used. These cells express constitutively the rat form of the ABCC6 transporter. Both cell line were classically cultured in DMEM medium culture containing 10% serum and penicillin/streptomycin (50 U/ml). Cultures were maintained in a water-saturated atmosphere of 5% CO₂/95% air at 37°C before use. Cells were used between passage 15 to 25. The measurements of extracellular PPi mediated by expression of rABCC6 were performed on cells cultured for 2 days until sub-confluence in the presence of puromycin (2µg/ml). The day of experiments, cultured medium was replaced by HBSS medium (red phenol free) containing 10% fetal bovine serum, glucose (4,5 g/l final), 10 mM HEPES, and 2mM glutamine. The HBSS supernatants were collected after 0.5, 1, 3 and 6 hours and centrifuged (5 min, 2000g, 4°C) to eliminate putative cells in suspension that can alter the PPi quantification. The samples were frozen at -80°C before use. For all experiments, the exact protein content in each well was measured using Bio-Rad protein assays (France).

### Human biological samples preparation

Biological samples from PXE patients were provided from the biobank of the national PXE Reference Center (MAGEC Nord, Angers University Hospital, Angers, France) as a part of the protocol for phenotyping the French PXE cohort (ClinicalTrials.gov Identifier: NCT01446380).

For PPi urinary quantification, urine samples were collected in the morning concomitantly to the blood samples after an overnight fasting period. Urine were collected in urine monovette collection tube. For enzymatic method, urine samples were diluted 16X in ultrapure water. For IC measurements, urine samples as well as calibration solutions were diluted 4X with distillated water.

For PPi plasma quantification, patient blood samples were collected in the morning after an overnight fasting period (venous punction) directly in Sodium citrate collection tube (Citrate 3.2% (2.7 ml, réf: 363048).The tubes were filled up to the limit or just below.

### Human plasma pre-analytic samples preparation

Citrate tubes were kept vertical on ice until centrifugation (1000-1200 g, 15 min, 4°C) to stack all blood cells (mainly erythrocytes and white cells). Plasma were collected and the level of hemolysis was estimated quantitatively by photometry measurement (absorbance at 546 nm for hemoglobin (Duranton et al., 2002 J. Physiol. 539, 847-855.). Less than 0.2% of RBC hemolysis was fixed as the maximal level to consider that free hemoglobin do not interfere with plasma ultra-filtration protocol and PPi quantification method. Plasmas were ultra-filtrated at 4°C using Amicon 0.5ml ultracel^{®} 50K filters (50 kDa cut-off; UFC505096, spin 20 min at 14000g) or either Centrisart filtration units (300.kDa cut-off, Sartorius 13279; spin 30 min at 2200g). Centrisart filters eliminate platelets and proteins with a molecular weight >of 300kDa from the plasmas. Amicon filters allows to remove platelets but also 95% of the proteins (ie, albumin) as indicated in the manufacturer instructions. Ultrafiltrates were clear and colorless and stored at -80° C. All the different steps of pre-analytical method were performed as fast as possible and successively to avoid degradation of PPi or blood cells lysis that can alter the measurements of the PPi in the ultrafiltrates.

### PPi quantification using enzymatic method: luciferin/luciferase assay

[PPi] is determined enzymatically using ATPsulfurylase to convert PPi into ATP in the presence of excess of APS (adenosine-5'-phosphosulfate). 2 solutions are needed. Solution A contains 0.1 U/ml ATPsulfurylase (New England Biolabs, MO394) and 80 µM APS (Sigma-Aldrich, A5508). Solution B containing 0.1 U/ml ATPsulfurylase, is treated 10 min at 90°C to inactivate ATPsulfurylase and then APS is added. 15 µl of each sample or standard are loaded in 2 different wells. In a well 5 µl of Solution A is added. In the second well 5 µl of Solution B is added. The plate is then incubated using a thermocycler (Mastercycler Eppendorf) for 30 min at 37 °C, and subsequently for 10 min at 90 °C to inactivate ATP sulfurylase. Generated ATP is quantified using the ATP Determination Kit ((ATPlite; PerkinElmer, Waltham, MA) according to the manufacturer's instructions. This ATP kit was chosen for its ability to generate over the time (at least 10 min) a stable and reliable luminescence with the use of recombinant luciferase protein. The luminescence (LUM) is measured 3 times over a 10 min period in white microplate on a microplate reader (Synergy HT, BioTek, US). PPi values are obtained by subtracting the basal ATP levels measured with solution B.

A special attention has been paid to any potential effect of the matrix (ions, proteins, or any substances). Therefore, for PPi determination in cell culture media, a standard curve of PPi was realized in the same colorless medium. For PPi determination in urine samples, the samples had to be diluted 16 times to calculate the sample concentration based on the calibration curve of PPi in water. Each plasma sample (5 µl) was spiked with 10 µl of increasing concentrations of PPi solution and PPi concentration of each plasma sample was determined using its own linear regression equation.

### Quantification of PPi using ion chromatography (IC)

Before IC analysis, 40 µl of all biological samples (cell culture medium, urine samples or patient plasma ultrafiltrates) were supplemented with EGTA (1mM final concentration) mixed and deproteinized using addition of acetonitrile (dilution 1:1 volume). Samples were strongly mixed and centrifuged at 12000g (10min at 4°C). The same protocol (addition of EGTA and acetonitrile) was also used for the quantification of the calibration samples (0.75, 1.5, 3, 5, 10µM of PPi in water or HBSS) to have in fine the same dilution factor and the same matrix composition.

Detection of PPi was performed using an ion chromatography Dionex ICS-5000 plus system (Thermo Scientific). The system included an autosampler, pumps, eluent generator and conductivity detectors. The system is equipped with an eluent generator (KOH, 500mM), a guard pre-column (AG11-HC, RFIC, 2X50mm) and an analytical anion column (lonPac AS-11-HC, 2X250mm, RFIC) for the detection of PPi. PPi measurement Protocol: 0.25 ml/min water debit, Sample injection loop: 10µl. elution protocol: 7mM of KOH (2min) followed by a linear increase period (20min) to reach 50 mM and a stable period (50mM, 4min) before a return period (7mM, 2min). (total time=28min).

PPi pics quantification were performed using chromaleon software (Thermo Scientific) by measuring surface area and were compared to the corresponding standard curve.

### Data analysis

Graphics and data analysis have been performed with Graphpad prism (Scientific Software). ANOVA t-test with Dunn's multiple comparisons test post-hoc test has been performed for plasmatic PPi concentration between the different groups of patients.

### Results

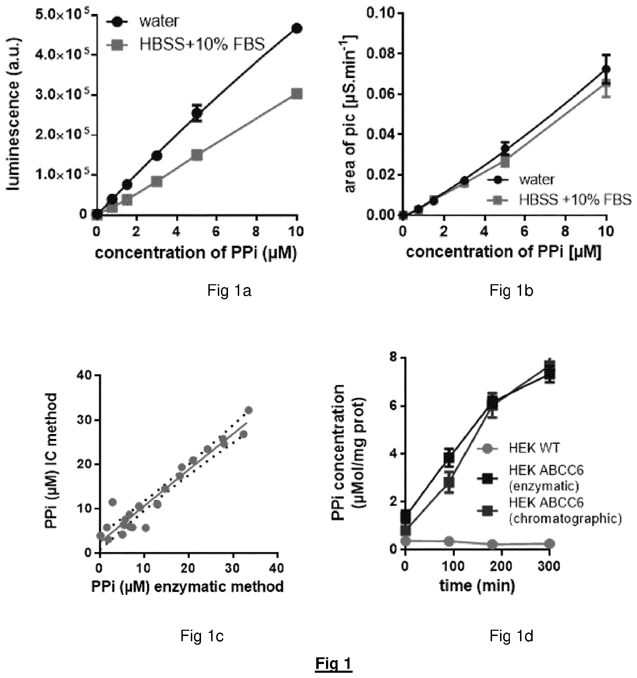

The measurement of PPi using enzymatic method is adapted from (Prosdocimo et al., 2009) and needs two enzymatic steps to convert PPi in ATP followed by the hydrolysis of the ATP to produce light using a bioluminescent reaction. In the first step, PPi is converted in ATP in the presence of adenosine 5' phosphosulfate (APS) and ATP sulfurylase. In the second step, the de novo-synthetized ATP is subsequently quantified using luciferin/luciferase bio-luminescent assay. Using this enzymatic method, a series of measurements were performed, where exogenous PPi (Na2PPi, 0.75, 1.5, 3, 5, 10 µM final concentrations) were added to ultrapure water or to a more physiological solution (HBSS supplemented with 10% serum). Increasing the amount of PPi in water or HBSS + 10% serum solution led to an increase in the luminescence in a linear manner for concentrations up to 10 µM. The composition of the medium strongly influenced the slope of the dose-response curves (Fig. 1a).

Therefore, the composition of the medium can alter the ATP-sulfurylase or luciferin/luciferase reactions and thus generate errors in the determination of the PPi concentrations (matrix effect). This is particularly the case with biological samples (urines, culture media, plasmas...) that exhibit different ionic or proteins compositions.

Next, the PPi concentrations was measured in the same samples using an ionic chromatographic (IC) method. Measurements of PPi concentration were performed in the same solutions as previously used for enzymatic experiments. IC method allows to detect significant peaks (retention time around 23 min) which exhibited an increase of their surfaces proportional to the increase of the PPi concentrations. Calculation of the peaks areas (µs.min-1) measured for the different concentrations of PPi are not significantly different between the 2 solutions (Fig. 1b) suggesting that the composition of the matrix had no effect on the PPi concentration using this IC technique.

To compare the values obtained with the 2 methods (enzymatic and IC) in the context of different biological fluids, first the level of extracellular PPi was quantified in the supernatant of cultured cells (culture medium). HEK293-ABCC6 cells represent a good model since it has been previously demonstrated that over-expression of the ABCC6 protein induced a marked and significant increase of PPi in the supernatant after few hours of culture. Supernatants were obtained from 2 different cell lines: WT HEK293 cells or HEK293 cells expressing stable rat ABCC6 transporter (kindly provided by Pr. Van de Wetering K.). At time 0, culture medium was replaced by a HBSS medium containing 10% serum and supernatants were collected after 0.5, 1, 3 and 6 hours and PPi concentrations were determined simultaneously using both the enzymatic and IC methods. With both methods, the calibration PPi solutions were prepared in the same culture medium (HBSS + 10% serum). Values are normalized to protein content measured in each well and are presented in Fig.1d. In the supernatants of the WT HEK293 cells, no significant increase in PPi concentration was detected with both techniques and for all the time points of the kinetic. By contrast, the PPi concentration increased significantly with time of incubation when measured in the supernatants of the HEK293-ABCC6 cells and the PPi values obtained with both techniques are almost similar for each time point of the kinetic. Altogether, this experiment confirms that the methods we used to quantify PPi concentrations in cell culture samples are reliable and give comparable results.

Both methods were next used in order to quantify PPi in human urine samples. PPi quantification was performed with both methods from 25 independent urine samples. For both methods, a dose response curve was performed using ultrapure distillated water supplemented with increasing concentration of exogenous PPi (from 0 to 10µM for enzymatic method and up to 50µM for the IC method). Urine samples were diluted in ultrapure water to limit the putative matrix effects observed previously using enzymatic method. As a result, the values measured with both methods were compared and fitted with a linear regression (Fig. 1c). A wide distribution of the urinary PPi concentrations was observed in the samples, from <1µM up to > 30µM. Fitting the PPi concentrations measured with both methods reveals a Spearman correlation of r=0.88 (p<0.0001). Bland-Altman analysis for comparison of the values obtained with the two methods reveals no significant bias suggesting that the methods are reliable within the PPi range and the experimental conditions used in this study.

Both techniques are then combined in order to quantify PPi in human plasma samples. A specific pre-analytic preparation of the samples was performed prior to determination of PPi concentration in human plasma.

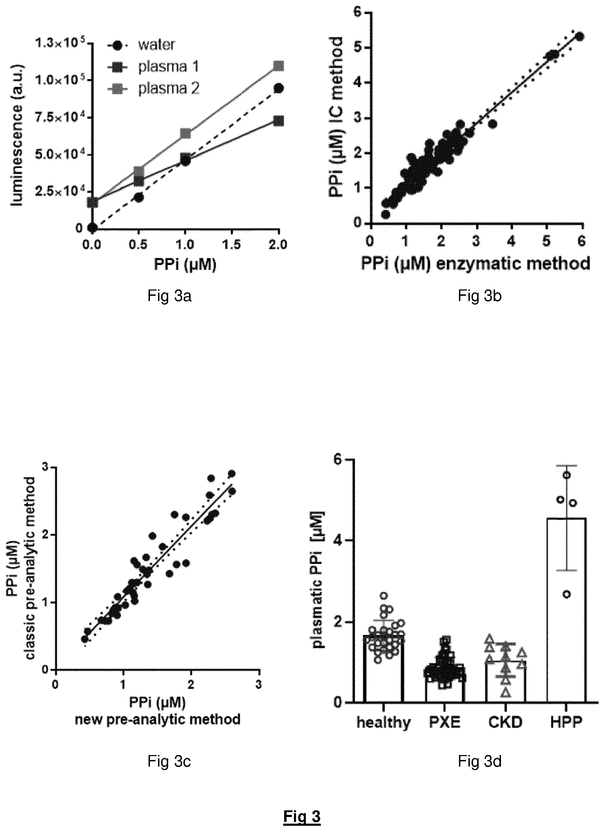

The freshly drawn human plasmas was exposed to an ultrafiltration procedure using 50 kDa cut-off filters to eliminate 95% of the proteins and all the putative cells in suspension (this centrifugation step with those filters can be performed with conventional bench centrifuge and strongly facilitates the way that samples are technically handled in our clinical facilities). The ultra-filtrated plasmas were then spiked with increasing concentrations of exogenous PPi (0, 0.5, 1 and 2 µM) to eliminate the influence of variable plasmas compositions between different donors (matrix effect). Fig. 3a illustrated this situation and showed the linear regression fitted for 2 different spiked ultrafiltrated plasmas and a calibrating PPi water solution. As a result, it was observed that the slope of plasma 2 (obtained by linear regression) is the same than the slope of the water solution, but the slope of plasma 1 is different.

Those results confirmed that measurement of PPi in plasma sample using enzymatic approach instead of using a classical PPi/water calibration curve, should preferably be done by calculating the slope of the linear regression of each spiked plasma. The values of PPi measured simultaneously in the same ultrafiltrate with both enzymatic and IC methods from 80 plasma samples (80 different donors) were next compared. The use of both methods to quantify PPi concentration in the same plasma sample allows to cross-control the values measured and to strengthen the accuracy of the measurements. A variation of more than 15% between the PPi values obtained with both methods was considered not acceptable and measurements have to be redone. Over the 80 paired measurements performed initially, 10 were not reaching the standards that were fixed and PPi quantifications were performed a second time with each method. After this additional run of measurements, values of PPi were fitted (linear regression) for the 80 different samples (Fig 3b) and give a Spearman correlation of r=0.964 (p<0.0001). Bland-Altman analysis reveals no significant bias (p=0.237) suggesting that this combined method is reliable within the PPi range found in human ultra-filtrated plasmas.

Using this combined method strongly enhances the accuracy of the PPi quantification and allows to calculate a PPi concentration (averaging the values obtained from both methods) very close to the real circulating PPi concentration.

Initially, a classical pre-analytical treatment of blood samples as described (Jansen et al., 2014) was used: trisodium EDTA (1mM) was added to blood samples and centrifuged to collect plasma that were subsequently depleted of platelets by filtration through a 300kD mass cut-off filter. However, addition of EDTA strongly limit the use of IC method because EDTA exhibits a time of retention very close to the one of PPi excluding the use of IC method for PPi determination.

This classically used pre-analytic technique was compared to the technique (EDTA + 300kD cut-off filters versus no EDTA + 50kD cut-off filters) using the enzymatic method.

From the same blood sample, 2 different ultrafiltrates were prepared, using the 2 pre-analytical techniques and PPi concentration was measured in each ultrafiltrate. This procedure was performed with 42 plasma samples drawn from 42 different patients and PPi concentrations were compared and fitted with a linear regression (Fig. 3c). A Spearman correlation of r=0.9476 (with p<0.0001) was observed between the PPi concentrations measured in the ultrafiltrates obtained from both pre-analytic methods. Bland-Altman analysis reveals no significant bias (p=0.137) suggesting that both pre-analytic methods are reliable within the PPi range used in this study. The 50 kD mass cut-off filtering technique without pre addition of EDTA to blood samples was chosen as the reference pre-analytic technique to be able to dose PPi with IC method and enzymatic one..

Finally, the combined method (enzymatic and IC) for PPi quantification with the new preanalytic method was applied in plasma samples obtained from patients suffering from inherited or acquired diseases known to alter the level of plasma PPi (Fig. 3d). Plasma PPi levels from these different groups was tested and compared to a group of healthy subjects. Patients comprise a group affected with Pseudoxanthoma elasticum (PXE) a disorder characterized by ectopic calcification and low PPi plasma levels. They also comprise hemodialyzed patients (HD) with chronic kidney disease under permanent renal replacement therapy and patients with hypophosphatasia (HPP), a rare inherited disorder that affects the development of bones and other mineralized tissues.

PPi concentration was 1.64±0.37 µM [1.07-2.65, n=34] in the healthy group and significantly lower in PXE patients (0.892±0.35 µM [0.43-2.07, n=36]) and 1.05±0.404 µM [0.26-1.59, n=10] for HD patients. By contrast, the PPi concentration of the 4 patients harboring the HPP inherited disorder were expectedly higher with a mean of 4.48+1.31 µM [2.58-5.59]. Altogether, the mean value of PPi measured in PXE patients is ~50% lower than the value in healthy patients. This result is in accordance with the literature

The reliable and reproducible method by coupling 2 different technical approaches (enzymatic and IC) is allowing to measure precisely the PPi concentrations of various biological fluids. For plasma PPi quantification, this combined method is associated to a novel pre-analytic method that strongly facilitates the way samples are technically handled.

## Claims

1. A method for determining the level of inorganic pyrophosphate (PPi) in a biological sample, comprising
a) Measuring the concentration of PPi in a first fraction of the biological sample, using an assay based on enzymatic reaction
b) Measuring the concentration of PPi in a second fraction of the biological sample, using an assay based on ionic chromatography
c) Comparing the value measured in step a) and the value measured in step b), and assessing if the difference is above a pre-determined threshold
d) If the difference between the values measured respectively in step a) and in step b) is lower than or equal to the pre-determined threshold, the concentration of PPi in the biological sample is determined as the mean of said values measured in step a) and in step b), and if the difference between the values measured respectively in step a) and in step b) is greater than the pre-determined threshold, steps a) to c) are repeated on new fractions of the biological sample.

2. The method according to claim 1, wherein the pre-determined threshold is equal to 15%.

3. The method according to claim 1, wherein the pre-determined threshold is equal to 10%.

4. The method according to anyone of claims 1 to 3, wherein the enzymatic assay of step a) comprises the step of
i) converting PPi to ATP by a reaction catalyzed by ATP sulfurylase
ii) producing light from ATP using luciferase in presence of luciferin
iii) detecting the bioluminescence produced as a measure of PPi in the sample.

5. The method according to anyone claims 1 to 4, wherein in step a), a calibration curve is first established, by adding increasing concentrations of exogenous PPi to fractions of the biological sample, and performing the assay based on enzymatic reaction on said fractions containing exogenous PPi.

6. The method according to anyone of claims 1 to 5, wherein in step b), a calcium chelating agent is added to the fraction of the biological sample prior to performing the ionic chromatography.

7. The method according to claim 6, wherein the calcium chelating agent is EGTA.

8. The method according to anyone of the preceding claims, wherein the biological sample is selected from blood, plasma, urine, saliva, synovial fluid, CSF and cell culture medium.

9. The method according to anyone of the preceding claims, comprising the pre-treatment of the biological sample to remove or reduce the presence of contaminants selected from proteins, lipids, cells and cell fragments, prior to performing step a).

10. The method according to anyone of the preceding claims, comprising the pre-treatment of the biological sample by a method selected from ultrafiltration and centrifugation.

11. The method according to anyone of claims 4 to 10, wherein in step a), the bioluminescence produced by the basal ATP level in the biological sample is subtracted from the bioluminescence measured by adding ATP sulfurylase.

12. The method according to anyone of the preceding claims, wherein in step a), a recombinant luciferase is used to produce bioluminescence.

13. An *in vitro* method for detecting a condition associated with deregulated inorganic pyrophosphate level in an individual, comprising submitting a biological sample from the said individual to a method according to anyone of the preceding claims for determining the level of inorganic pyrophosphate (PPi) in said biological sample.

## Patentansprüche

1. Verfahren zur Bestimmung des Gehalts an anorganischem Pyrophosphat (PPi) in einer biologischen Probe, umfassend
a) Messen der PPi-Konzentration in einer ersten Fraktion der biologischen Probe unter Verwendung eines auf enzymatischer Reaktion basierenden Assays
b) Messen der PPi-Konzentration in einer zweiten Fraktion der biologischen Probe unter Verwendung eines auf Ionenchromatographie basierenden Assays
c) Vergleichen des in Schritt a) gemessenen Werts und des in Schritt b) gemessenen Werts und Beurteilen, ob die Differenz über einem vorbestimmten Schwellenwert liegt
d) wenn die Differenz zwischen den in Schritt a) bzw. in Schritt b) gemessenen Werten kleiner als der oder gleich dem vorbestimmte(n) Schwellenwert ist, wird die PPi-Konzentration in der biologischen Probe als Mittelwert der in Schritt a) und in Schritt b) gemessenen Werte bestimmt, und wenn die Differenz zwischen den in Schritt a) bzw. in Schritt b) gemessenen Werten größer als der vorbestimmte Schwellenwert ist, werden die Schritte a) bis c) an neuen Fraktionen der biologischen Probe wiederholt.

2. Verfahren nach Anspruch 1, wobei der vorbestimmte Schwellenwert gleich 15 % ist.

3. Verfahren nach Anspruch 1, wobei der vorbestimmte Schwellenwert gleich 10 % ist.

4. Verfahren nach einem der Ansprüche 1 bis 3, wobei der enzymatische Assay von Schritt a) den Schritt umfasst, bei dem
i) PPi durch eine durch ATP-Sulfurylase katalysierte Reaktion in ATP überführt wird
ii) Licht aus ATP unter Verwendung von Luciferase in Gegenwart von Luciferin erzeugt wird
iii) die erzeugte Biolumineszenz als Maß für PPi in der Probe nachgewiesen wird.

5. Verfahren nach einem der Ansprüche 1 bis 4, wobei in Schritt a) zunächst eine Eichkurve erstellt wird, indem Fraktionen der biologischen Probe mit steigenden Konzentrationen von exogenem PPi versetzt werden und der auf enzymatischer Reaktion basierende Assay an den exogenes PPi enthaltenden Fraktionen durchgeführt wird.

6. Verfahren nach einem der Ansprüche 1 bis 5, wobei in Schritt b) vor dem Durchführen der Ionenchromatographie ein Calciumchelatbildner zu der Fraktion der biologischen Probe gegeben wird.

7. Verfahren nach Anspruch 6, wobei es sich bei dem Calciumchelatbildner um EGTA handelt.

8. Verfahren nach einem der vorhergehenden Ansprüche, wobei die biologische Probe aus Blut, Plasma, Urin, Speichel, Synovialflüssigkeit, CSF und Zellkulturmedium ausgewählt ist.

9. Verfahren nach einem der vorhergehenden Ansprüche, umfassend die Vorbehandlung der biologischen Probe zur Entfernung oder Verringerung des Vorliegens von Kontaminanten, die aus Proteinen, Lipiden, Zellen und Zellfragmenten ausgewählt sind, bevor Schritt a) durchgeführt wird.

10. Verfahren nach einem der vorhergehenden Ansprüche, umfassend die Vorbehandlung der biologischen Probe mit einem Verfahren, das aus Ultrafiltration und Zentrifugation ausgewählt ist.

11. Verfahren nach einem der Ansprüche 4 bis 10, wobei in Schritt a) die durch das ATP-Grundniveau in der biologischen Probe erzeugte Biolumineszenz von der durch Zugabe von ATP-Sulfurylase gemessenen Biolumineszenz subtrahiert wird.

12. Verfahren nach einem der vorhergehenden Ansprüche, wobei in Schritt a) eine rekombinante Luciferase zum Erzeugen von Biolumineszenz verwendet wird.

13. In-vitro-Verfahren zum Nachweisen eines Leidens in Zusammenhang mit einem deregulierten Spiegel von anorganischem Pyrophosphat bei einem Individuum, umfassend Unterziehen einer biologischen Probe von dem Individuum einem Verfahren nach einem der vorhergehenden Ansprüche zur Bestimmung des Spiegels von anorganischem Pyrophosphat (PPi) in der biologischen Probe.

## Revendications

1. Procédé pour déterminer le taux de pyrophosphate inorganique (PPi) dans un échantillon biologique, comprenant
a) mesure de la concentration en PPi dans une première fraction de l'échantillon biologique, à l'aide d'un dosage basé sur une réaction enzymatique
b) mesure de la concentration en PPi dans une seconde fraction de l'échantillon biologique, à l'aide d'un dosage basé sur la chromatographie ionique
c) comparaison de la valeur mesurée à l'étape a) et de la valeur mesurée à l'étape b), et évaluation si la différence est supérieure à un seuil prédéterminé
d) si la différence entre les valeurs mesurées respectivement à l'étape a) et à l'étape b) est inférieure ou égale au seuil prédéterminé, la concentration en PPi dans l'échantillon biologique est déterminée comme la moyenne desdites valeurs mesurées à l'étape a) et à l'étape b), et si la différence entre les valeurs mesurées respectivement à l'étape a) et à l'étape b) est supérieure au seuil prédéterminé, les étapes a) à c) sont répétées sur de nouvelles fractions de l'échantillon biologique.

2. Procédé selon la revendication 1, dans lequel le seuil prédéterminé est égal à 15 %.

3. Procédé selon la revendication 1, dans lequel le seuil prédéterminé est égal à 10 %.

4. Procédé selon l'une quelconque des revendications 1 à 3, dans lequel le dosage enzymatique de l'étape a) comprend l'étape de
i) conversion de PPi en ATP par une réaction catalysée par l'ATP sulfurylase
ii) production de lumière à partir de l'ATP en utilisant la luciférase en présence de luciférine
iii) détection de la bioluminescence produite comme mesure de PPi dans l'échantillon.

5. Procédé selon l'une quelconque des revendications 1 à 4, dans lequel, dans l'étape a), une courbe d'étalonnage est d'abord établie, en ajoutant des concentrations croissantes de PPi exogène à des fractions de l'échantillon biologique, et en réalisant le dosage basé sur une réaction enzymatique sur lesdites fractions contenant du PPi exogène.

6. Procédé selon l'une quelconque des revendications 1 à 5, dans lequel, dans l'étape b), un agent chélatant du calcium est ajouté à la fraction de l'échantillon biologique avant d'effectuer la chromatographie ionique.

7. Procédé selon la revendication 6, dans lequel l'agent chélatant du calcium est l'EGTA.

8. Procédé selon l'une quelconque des revendications précédentes, dans lequel l'échantillon biologique est choisi parmi le sang, le plasma, l'urine, la salive, le liquide synovial, le LCR et un milieu de culture cellulaire.

9. Procédé selon l'une quelconque des revendications précédentes, comprenant le prétraitement de l'échantillon biologique pour éliminer ou réduire la présence de contaminants choisis parmi les protéines, les lipides, les cellules et les fragments cellulaires, avant de réaliser l'étape a).

10. Procédé selon l'une quelconque des revendications précédentes, comprenant le prétraitement de l'échantillon biologique par un procédé choisi parmi l'ultrafiltration et la centrifugation.

11. Procédé selon l'une quelconque des revendications 4 à 10, dans lequel, dans l'étape a), la bioluminescence produite par le taux basal d'ATP dans l'échantillon biologique est soustraite de la bioluminescence mesurée par ajout d'ATP sulfurylase.

12. Procédé selon l'une quelconque des revendications précédentes, dans lequel dans l'étape a), une luciférase recombinante est utilisée pour produire de la bioluminescence.

13. Procédé *in vitro* pour détecter une affection associée à un taux de pyrophosphate inorganique dérégulé chez un individu, comprenant la soumission d'un échantillon biologique dudit individu à un procédé selon l'une quelconque des revendications précédentes pour déterminer le taux de pyrophosphate inorganique (PPi) dans ledit échantillon biologique.
